# EUROPEAN PATENT APPLICATION

(11) **EP 3 629 130 A1**
(43) Date of publication of application: **01.04.2020**
(21) Application number: 18212823.1
(22) Date of filing: 15.12.2018
(51) Int. Cl.: G06F 3/01, A61B 5/00, G06N 3/00, G06N 20/00, A63F 13/00

(54) **ARTIFICIAL INTELLIGENCE SYSTEM AND INTERACTIVE RESPONDING METHOD**

(30) Priority: 25.09.2018 US 201816140552
(71) Applicant: XRSpace CO., LTD., Taoyuan City 330 (TW)
(72) Inventor: Chou, Peter, 100 Taipei City (TW); Chu, Feng-Seng, 235 New Taipei City (TW); Lee, Cheng-Wei, 205 Keelung City (TW); Lai, Yu-Chen, 333 Taoyuan City (TW); Wang, Chuan-Chang, 116 Taipei City (TW)
(74) Representative: Straus, Alexander

(57) **Abstract**

An embodiment of the invention relates to an artificial intelligence system comprising at least two servers used to generate instructions for a non-player character (NPC) to interact with a user depending on whether the user is within 1 metre of the NPC in the virtual world and configured to determine a plurality of interactions in response to the received input data related to an action, a facial expression, a gaze, a text, a speech, a gesture, an emotion or a movement detected from the user.

## Description

### Field of the Invention

The present disclosure relates to an artificial intelligence (AI) system and interactive responding method, and more particularly, to an AI system and interactive responding method capable of fusing a game AI with an interactive Al.

### Background of the Invention

With the advancement and development of technology, the demand of interactions between a computer system and a user is increased. Human-computer interaction technology, e.g. somatosensory games, a virtual reality (VR) environment, an augmented reality (AR) environment, a mixed reality (MR) environment and an extended reality (XR) environment, becomes popular because of its physiological and entertaining function. In the above stated environments, a non-player character (NPC) is created to help a user or player characters, such as an NPC avatar. The NPC is embedded with a game artificial intelligence (AI) which enables the NPC to interact with the user or the player characters, such as, replying or reacting to simple messages or questions from the user or the player characters, but the current NPC can only reply simple questions or mechanical responses, which is restricted to texts or words in the above stated environments.

However, since the user may express in all kinds of methods in the environments, e.g. speech, facial expression, body movement and gesture. Under this situation, the NPCs in the environments, which are not capable of being interactive with the user or being a human-like NPC, affects the user experience.

### Summary of the Invention

It is therefore an objective of the present disclosure to provide an artificial intelligence system and interactive responding method to improve the NPC in the somatosensory games or VR/AR/MR/XR environments to be more interactive to the user, the user avatar or the player characters and provide a better user experience.

This is achieved by an artificial intelligence system according to the independent claim 1 or by an interactive responding method according to the independent claim 6 here below. The dependent claims pertain to corresponding further developments and improvements.

As will be seen more clearly from the detailed description following below, the claimed artificial intelligence system comprises a first server configured to receive first input data and to determine whether the first input data conform to any one of a plurality of variation conditions, and generate a second input data when the first input data conform to any one of the plurality of variation conditions ; and a second server communicated with the first server and configured to determine a plurality of interactions in response to the received second input data; wherein the first input data are related to an action, a facial expression, a gaze, a text, a speech, a gesture, an emotion or a movement detected from a user.

In another aspect, the claimed interactive responding method, for an artificial intelligence (AI) system, comprises a first server receiving first input data and determining whether the first input data conform to any one of a plurality of variation conditions and generating a second input data when the first input data conform to any one of the plurality of variation conditions; and a second server determining a plurality of interactions in response to in response to the received second input data; wherein the first input data are related to a an action, a facial expression, a gaze, text, a speech, a gesture, an emotion or a movement detected from a user.

### Brief Description of the Drawings

FIG. 1 is a schematic diagram of an artificial intelligence system according to an embodiment of the present disclosure.
FIG. 2 is a schematic diagram of an operation process of the artificial intelligence system according to an embodiment of the present disclosure.
FIG. 3 is a schematic diagram of an interactive responding process according to an embodiment of the present disclosure.

### Detailed Description

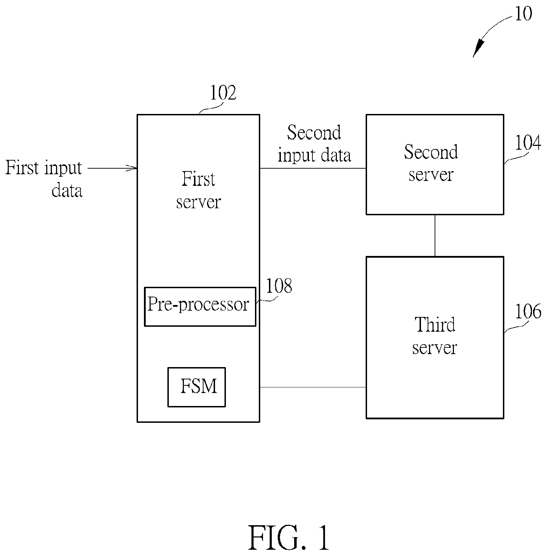

Please refer to FIG. 1, which is a schematic diagram of an artificial intelligence (AI) system 10 according to an embodiment of the present disclosure. The AI system 10 includes a first server 102, a second server 104 and a third server 106. The first server 102 is configured to receive first input data from a user and to determine whether the first input data conform to any one of a plurality of variation conditions or not. In addition, the first server 102 generates a second input data when the first input data conform to any one of the plurality of variation condition. In an embodiment, the first server 102 may be a game AI server, which receives information detected from the user in somatosensory games or VR/AR/MR/XR environments. The received information of the first server 102 may be actions, facial expressions, gazes, texts, speeches, messages, body gestures or body movements generated by the user, and then, the first server 102 determines whether the information generated by the user conforms to the variation conditions or not. The second server 104 is configured to communicate with the first server 102 and to determine a plurality of interactions in response to the received second input data from the user. Notably, the first input data and the second input data might be the same. In an embodiment, the second server 104 may be a Chatbot server, which determines the interactions in response to the actions, facial expressions, gazes, texts, speeches, messages, body gestures or body movements detected from the user. After the interactions are determined, the first server 102 displays a plurality of actions corresponding to the interactions via a non-player character (NPC). In an embodiment, the first server 102 may transmit a signal corresponding to an action, a facial expression, a gaze, a text, a speech, a gesture, an emotion or a motion to the NPC. The NPC may display the action, the facial expression, the gaze, the text, the speech, the gesture, the emotion or the motion corresponding to the signal and thereby present on a front end display of the AI system 10. The third server 106 may include a data application programming interface (API) server to retrieve and store a plurality of the states of the user and the NPC for the interactions in response to the first input data. In detail, the third server 106 stores the states of the user corresponding to the interactions displayed via the non-player character, such as a friendship value between the user and NPC, a sport preference value of the user or a happiness value of the NPC. In an embodiment, the third server 106 may store that the user prefers soccer to basketball into a database. Therefore, the AI system 10 of the present disclosure coordinates the game AI server (i.e. the first server 102) with the Chatbot server (i.e. the second server 104) to interact with the user via the NPC with speeches, facial expressions, body movements and gestures.

Generally, an action, a facial expression, a gaze, a text, a speech, a gesture, an emotion or a motion generated by the user in the somatosensory games or VR/AR/MR/XR environments is corresponded or related to an action, a facial expression, a gaze, a text, a speech, a gesture, an emotion or a motion detected by at least a sensor and from the user in the real world.

In some embodiment, the user cannot see his/her own avatar or player characters in the somatosensory games or VR/AR/MR/XR environments. The user can only see the NPCs and/or other people's avatars. Therefore, it is possible that the user feel his/her own avatar does not exist and he/she is actually interact with the NPCs and/or other people's avatars in the real world.

The examples mentioned above briefly explain that the AI system 10 of the present disclosure provides a more intelligent and interactive method to communicate with the user in the somatosensory games or VR/AR/MR/XR environments. Notably, those skilled in the art may make proper modifications. For example, the first server 102 and the second server 104 are not limited to be implemented by the game AI server and the Chatbot server, and other kinds of servers. For another example, the first server 102 may use, analyze and/or understand a detection result as the first input data (or as a part of the first input data), or use an action, a facial expression, a gaze, a text, a speech, a gesture, an emotion or a motion generated by a player characters or a user avatar as the first input data (or as another part of the first input data).

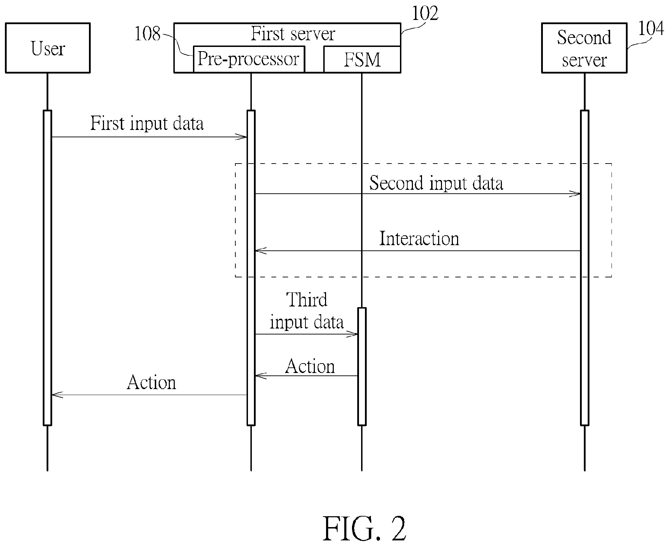

In detail, the first server 102 includes a pre-processor 108 and a finite state machine (FSM). The pre-processor 108 generates third input data according to the first input data and the interactions generated by the second server 104. The FSM transits among a plurality of states according to the third input data, wherein the states correspond to the interactions. More specifically, please refer to FIG. 2, which is a schematic diagram of an operation process of the AI system 10 according to an embodiment of the present disclosure. The first server 102 receives the first input data generated by the user, such as speeches, movements or gestures, and then, the first server 102 determines whether the first input data conform to any one of the variation conditions, such as, whether a distance between the user and the NPC in the somatosensory games or VR/AR/MR/XR environments is less than 1 meter. When the variation condition is satisfied, i.e. the distance between the user and the NPC is less than 1 meter, the first server 102 transfers the second input data to the second server 104. And the second server 104 determines the interactions in response to the first input data. In an embodiment, the third server 106 memorizes the interactions corresponding to the first input data determined by the second server 104, and updates the interactions with the first server 102 and the second server 104. After the pre-processor 108 receives the interactions corresponding to the first input data form the second server 104, the pre-processor 108 generates the third input data. The FSM of the first server 102 further generates the corresponding actions and controls the NPC to display the corresponding actions to the user when the state of the FSM is changed according to the third input data, e.g. an emotion state of the FSM is changed from happy to angry. Moreover, the FSM updates the states of the FSM to the first server 102. In this way, the user may interact with the NPC in a more interactive way in the somatosensory games or VR/AR/MR/XR environments. Notably, the first input data from the user are related to a text, a speech, a movement, a gesture or an emotion generated by the user, and not limited thereto.

In another embodiment, when the first input data made by the user do not conform to any one of the variation conditions, the FSM of the first server 102 may directly evaluate the first input data and generate corresponding actions thereto. More specifically, when the user asks the NPC essential questions, such as commands, self-introduction or road direction, the FSM of the first server 102 may directly look up the third server 106 for reference and determine the corresponding actions to the questions. In an embodiment, when the user asks the AI system 10 to play music or video, the FSM of the first server 102 may directly generate the corresponding actions and play music or video. In this example, the first server 102 does not transfer the first input data to the second server 104 for further determination of interactions, since none of the variation conditions is satisfied.

Since the first input data are related to the text, speech, movement, gesture or emotion of the user in the real world, the second server 104 of the AI system 10 may control the actions or the emotions displayed via the NPC accordingly. In a usage scenario of the virtual reality environment, when the user actively walks to the NPC and chat or when the user stays still and stares at the NPC for over 6 seconds, the pre-processor 108 determines that the NPC actively walks to the user and chat according to the states of the FSM. Alternatively, when the user walks by the NPC for more than 3 times, the pre-processor 108 determines that the NPC actively walks to the user and asks the user for a reason accordingly, therefore the user may feel the NPCs actually come in front and ask a question in the real world.

In addition to the actions stated above, the FSM may determine actions with the NPC by gestures, movements or emotions. In an example, when the user is too far from the NPC, the FSM may determine the action about asking the user to stay closer to the NPC with a movement of waving hands, therefore the user may feel the NPCs actually wave their hands in the real world.

In another example, when the user attempts to touch or contact the NPC in the virtual reality environment, the FSM may determine the actions of stopping the user with a speech or shunning the user with anger. Or, in still another example, the FSM may determine the actions of covering a mouth of the NPC when agreeing with the user.

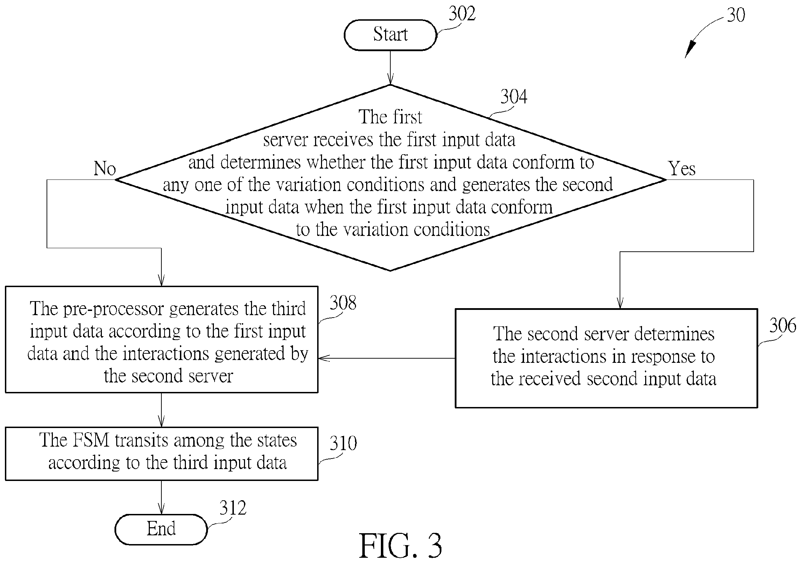

Based on different applications and design concepts, the AI system 10 of the present disclosure may be implemented in all kinds of methods. Furthermore, the operating process of the AI system 10 may be concluded to an interactive responding process 30 as shown in FIG. 3, which includes the following steps:
Step 302: Start.
Step 304: The first server 102 receives the first input data and determines whether the first input data conform to any one of the variation conditions and generates the second input data when the first input data conform to the variation conditions. If yes, execute step 306; if no, execute step 308.
Step 306: The second server 104 determines the interactions in response to the received second input data.
Step 308: The pre-processor 108 generates the third input data according to the first input data and the interactions generated by the second server 104.
Step 310: The FSM transits among the states according to the third input data.
Step 312: End.

The details of the interactive responding process 30 may be referred to the above mentioned embodiments of the AI system 10 and are not narrated herein for brevity.

Notably, the embodiments stated above illustrate the concept of the present disclosure, those skilled in the art may make proper modifications accordingly, and not limited thereto. For example, the variation conditions may be varied or be adjusted according to indications of a user or a manufacturer, or settings of a computer system, the interactions or the actions stored in a database of the third server, and not limited thereto, which all belongs to the scope of the present disclosure.

In summary, the present disclosure provides an artificial intelligence system and interactive responding method to improve the NPC in the somatosensory games or VR/AR/MR/XR environments to be more interactive to the user, the user avatar or the player characters, such that the NPC may interact with the user with involvements of speeches, body gestures and emotions and provide a better user experience.

## Claims

1. An artificial intelligence, abbreviated to Al, system (10), **characterized by** comprising:
a first server (102) configured to receive first input data and to determine whether the first input data conform to any one of a plurality of variation conditions, and generate a second input data when the first input data conform to any one of the plurality of variation conditions; and
a second server (104) communicated with the first server (102) and configured to determine a plurality of interactions in response to the received second input data;
wherein the first input data are related to an action, a facial expression, a gaze, a text, a speech, a gesture, an emotion or a movement detected from a user.

2. The AI system (10) of claim 1, **characterized in that** the first server (102) comprises:
a pre-processor (108) configured to generate third input data according to the first input data and the plurality of interactions generated by the second server (104); and
a finite state machine, abbreviated to FSM, coupled to the pre-processor (108) and configured to transit among a plurality of states according to the third input data;
wherein the plurality of states correspond to a plurality of actions.

3. The AI system (10) of claim 2, **characterized in that** the first server (102) is configured to transmit a signal corresponding to an action, a facial expression, a gaze, a text, a speech, a gesture, an emotion or a motion to a front end display to display the action, the facial expression, the gaze, the text, the speech, the gesture, the emotion or the motion via a non-player character, abbreviated to NPC.

4. The AI system (10) of claim 2, **characterized by** further comprising:
a third server (106) communicated with the second server (104) and configured to retrieve and store the plurality of actions, interactions, a plurality of non-player character, abbreviated to NPC, states and a plurality of user states in response to the first input data.

5. The AI system (10) of claim 4, **characterized in that** the third server (106) updates the plurality of actions, the interactions, the NPC states and the user states in response to the first input data with the first server (102) and the second server (104).

6. An interactive responding method (30), for an artificial intelligence, abbreviated to Al, system, **characterized by** comprising:
a first server (102) receiving first input data and determining whether the first input data conform to any one of a plurality of variation conditions and generating a second input data when the first input data conform to any one of the plurality of variation conditions (304); and
a second server (104) determining a plurality of interactions in response to in response to the received second input data (306);
wherein the first input data are related to a an action, a facial expression, a gaze, text, a speech, a gesture, an emotion or a movement detected from a user.

7. The interactive responding method of claim 6, **characterized by** comprising:
a pre-processor (108) generating third input data according to the first input data and the plurality of interactions generated by the second server (104) (308); and
a finite state machine (FSM) transiting among a plurality of states according to the third input data (310);
wherein the plurality of states correspond to a plurality of actions.

8. The interactive responding method of claim 7, **characterized in that** the first server (102) is configured to transmit a signal corresponding to an action, a facial expression, a gaze, a text, a speech, a gesture, an emotion or a motion to a front end display to display the action, the facial expression, the gaze, the text, the speech, the gesture, the emotion or the motion via a non-player character, abbreviated to NPC.

9. The interactive responding method of claim 7, **characterized by** comprising:
a third server (106) retrieving and storing the plurality of actions, interactions, a plurality of NPC states and a plurality of user states in response to the first input data.

10. The interactive responding method of claim 9, **characterized in that** the third server (106) updates the plurality of actions, the interactions, the NPC states and the user states in response to the first input data with the first server (102) and the second server (104).

## Amended claims

### Amended claims in accordance with Rule 137(2) EPC.

1. A reality interactive responding system (10), **characterized in that** the reality interactive responding system (10) comprises:
a first server (102) configured to receive first input data and to determine whether the first input data conform to any one of a plurality of variation conditions, and generate a second input data when the first input data conform to any one of the plurality of variation conditions; and
a second server (104) communicated with the first server (102) and configured to determine a plurality of interactions in response to the received second input data;
wherein the first input data are related to an action, a facial expression, a gaze, a text, a speech, a gesture, an emotion or a movement detected from a user.

2. The reality interactive responding system (10) of claim 1, **characterized in that** the first server (102) comprises:
a pre-processor (108) configured to generate third input data according to the first input data and the plurality of interactions generated by the second server (104); and
a finite state machine, abbreviated to FSM, coupled to the pre-processor (108) and configured to transit among a plurality of states according to the third input data;
wherein the plurality of states correspond to a plurality of actions.

3. The reality interactive responding system (10) of claim 2, **characterized in that** the first server (102) is configured to transmit a signal corresponding to an action, a facial expression, a gaze, a text, a speech, a gesture, an emotion or a motion to a front end display to display the action, the facial expression, the gaze, the text, the speech, the gesture, the emotion or the motion via a non-player character, abbreviated to NPC.

4. The reality interactive responding system (10) of claim 2, **characterized by** further comprising:
a third server (106) communicated with the second server (104) and configured to retrieve and store the plurality of actions, interactions, a plurality of non-player character, abbreviated to NPC, states and a plurality of user states in response to the first input data.

5. The reality interactive responding system (10) of claim 4, **characterized in that** the third server (106) updates the plurality of actions, the interactions, the NPC states and the user states in response to the first input data with the first server (102) and the second server (104).

6. A reality interactive responding method (30), for a reality interactive responding system, **characterized in that** the reality interactive responding method (30) further comprises:
a first server (102) receiving first input data and determining whether the first input data conform to any one of a plurality of variation conditions and generating a second input data when the first input data conform to any one of the plurality of variation conditions (304); and
a second server (104) determining a plurality of interactions in response to in response to the received second input data (306);
wherein the first input data are related to an action, a facial expression, a gaze, a text, a speech, a gesture, an emotion or a movement detected from a user.

7. The reality interactive responding method of claim 6, **characterized by** comprising:
a pre-processor (108) generating third input data according to the first input data and the plurality of interactions generated by the second server (104) (308); and
a finite state machine (FSM) transiting among a plurality of states according to the third input data (310);
wherein the plurality of states correspond to a plurality of actions.

8. The reality interactive responding method of claim 7, **characterized in that** the first server (102) is configured to transmit a signal corresponding to an action, a facial expression, a gaze, a text, a speech, a gesture, an emotion or a motion to a front end display to display the action, the facial expression, the gaze, the text, the speech, the gesture, the emotion or the motion via a non-player character, abbreviated to NPC.

9. The reality interactive responding method of claim 7, **characterized by** comprising:
a third server (106) retrieving and storing the plurality of actions, interactions, a plurality of NPC states and a plurality of user states in response to the first input data.

10. The reality interactive responding method of claim 9, **characterized in that** the third server (106) updates the plurality of actions, the interactions, the NPC states and the user states in response to the first input data with the first server (102) and the second server (104).
